# EUROPEAN PATENT APPLICATION

(11) **EP 1 832 575 A1**
(43) Date of publication of application: **12.09.2007**
(21) Application number: 06425147.3
(22) Date of filing: 06.03.2006
(51) Int. Cl.: C07C 381/04, C07D 487/04, A61K 31/216, A61K 31/22, A61K 31/519

(54) **Anti-inflammatory agents**

(71) Applicant: Sulfidris S.r.l., 20131 Milan (IT)
(72) Inventor: Sparatore, Anna, 20146 Milan (IT); Del Soldato, Piero, 20052 Monza (IT); Santus, Giancarlo, 20146 Milan (IT)
(74) Representative: Parisi, Luigi

(57) **Abstract**

The present invention relates to novel thiosulfonates derivatives. The present invention also provides methods for preventing, treating and/or reducing inflammation-associated diseases in the cardiovascular, connective tissue, pulmonary, gastrointestinal, respiratory, urogenital, nervous or cutaneous systems as well as infective and tumoral diseases employing said compounds.

## Description

### Field of the invention

This invention relates to the field of new compounds that are derivatives of organic thiosulfonates whose main activity is in the inflammation area.

It is well known that inflammatory processes are a major event leading to the development of severe diseases such as arthritis, cancer, atherosclerosis, impotence, hypertension, Alzheimer, COPD (Chronic Obstuctive Pulmonary Disease), etc..

Conventional treatments are based also on the ability of the drugs to down-regulate, at least partially, the inflammatory pathogenetic component of the disease.

### Summary of the invention

This invention relates to novel compounds that comprise in their formula the thiosulfonate group and are useful for treating diseases and pathological conditions involving inflammation as important pathogenetic process.

The results have been surprising, in that not only the safety was dramatically improved but also the efficacy was sometimes increased.

This invention also relates to processes for preparing these compounds and to pharmaceutical compositions containing these compounds.

The thiosulfonates included in the present invention are organic compounds having the following general formula: R-S-SO₂-R'.

### Description of the invention

An object of the present invention are compounds of general formula:

D-X-Y-W (I)

wherein D is a drug linked to ~X-Y-W ;
X is zero, C=O, COOZ or CONH, wherein Z is a straight or branched C₁-C₄-alkyl;
Y is zero, -O-(CH₂)ₙ-O- or -OOC-(CH₂)ₙ-COO- ,wherein n is 2-10;
W is an organic thiosulfonate moiety having the following formula:

   R-S-SO₂-R' (II)

   wherein R is -(CH₂)ₙ₋COO~; -(CH₂)ₙ-O~; -(CH₂)ₙ-NH~; n = 1-10 linear or branched and it is linked to D-X-Y~; R', straight or branched, is an alkyl, such as methyl, ethyl, propyl, alkenyl, alkinyl, alkylaryl, alkenylaryl, alkinylaryl, arylalkyl, arylalkenyl, arylalkinyl or cycloalkyl, cycloalkenyl, cycloalkinyl, or aromatic and/or heterocyclic ring, all substituted or unsubstituted; and salts thereof.
As a further object of the present invention are pharmaceutical compositions comprising at least one compound according to general formula (I), and salts thereof, as an active ingredient, moreover, as a further object of the present invention, in combination with pharmaceutically acceptable adjuvant(s) or carrier(s).

According to the present invention it has been found that it is possible to link an organic thiosulfonate moiety W (of general formula (II)) to a pharmacologically active compound helpful for treating disorders in the cardiovascular, connective tissue, pulmonary, gastrointestinal, respiratory, urogenital, nervous or cutaneous systems, infective diseases and tumoral diseases. The resulting compounds have good bioavailability, increased safety and maintain good efficacy.

The parent drugs D (i.e. the drugs to which the thiosulfonate moiety is attached) in the present invention can be selected within a wide class of compounds, such as antitumorals such as ancitabine, capecitabine, valproic acid, NSAIDs i.e. selective and unselective cyclo-oxygenase (COX) inhibitors such as ketoprofen, flurbiprofen, suprofen, indobufen, etodolac, diclofenac, indomethacin, naproxen, acetyl salicylic acid, celecoxib, rofecoxib, nimesulide, valdecoxib, parecoxib, lumiracoxib, diflunisal, meloxicam, etc., analgesics such as paracetamol, capsaicin, steroids such as chenodeoxycholic acid, flunisolide, antibiotics such as bacilysin, bronchodilators such as albuterol, expectorants and mucolitic agents such as ambroxol, anti-asthma, antiallergic and anti-histaminic drugs such as epinastine, ACE-inhibitors such as captopril, β-blockers such as atenolol, drugs for vascular disorders suc as brovincamine, antidiabetics such as glibomuride, antiulcer such as omeprazole, arbaprostil, antihyperlipidemics such as fluvastatine, antibacterials such as amoxicilline, antivirals such as amantadine, cGMP phosphodiesterase inhibitors such as sildenafil, verdenafil, drugs against dementia such as mofegiline and the bone reabsorption such as alendronic acid.

When the compounds include at least one asymmetric carbon atom, the products can be used in racemic mixture or in form of single enantiomer.

In the present invention the parent compound is considered in its original form or in a proper modification to allow the chemical manipulation with the thiosulfonate group.

Also parent drugs able to release nitric oxide exogenously or endogenously are useful for the present invention.

The substances can be linked via different linking groups such as esters, amides, imides, sulfonamides, azo groups, carbamates, carbonates, anhydrides, acetals, thioacetals, etc. The thiosulfonate moiety can be also directly linked by an ionic bond to the drug as salt when X=Y=0.

Bifunctional linkers known to the expert in the field (such as ethyl, propyl, or butyl diols; diamines; hydroxy amines, etc.) can be optionally present when they are necessary to link the drug to the thiosulfonate group.

As a further object of the present invention are salts of the compounds of general formula (I) which are pharmaceutical acceptable salts of compounds of formula (I), such as for example salts with alkaline metals and alkaline earth metals, non-toxic amines and aminoacids, inorganic acids such as hydrochloric acid, phosphoric acid, etc. or organic acids such as fumaric acid, citric acid, tartaric acid, etc.

Salts of thiosulfonates such as, for example, S-(2-carboxyethyl) methanthiosulfonate, S-(2-aminoethyl) methanthiosulfonate with the different drugs above described, are also part of the present invention.

As a further object of the present invention are compounds of general formula (I), wherein the moiety W is selected from the thiosulfonate group comprising S-(2-carboxyethyl) methanthiosulfonate, S-(2-aminoethyl) methan-thiosulfonate and S-(2-hydroxyethyl) methanthiosulfonate.

As a further object of the present invention are compounds according to general formula (I) such as 2-acetoxybenzoic acid 2-methanesulfonylsulfanyl-ethyl ester, {4-[4-ethoxy-3-(1-methyl-7-oxo-3-propyl-6,7-dihydro-1*H*-pyrazolo[4,3-d]pyrimidin-5-yl)-benzenesulfonyl]-piperazin-1-yl}-acetic acid 2-methanesulfonylsulfanyl-ethyl ester, [3-(2,6-dichloro-phenylamino)-phenyl]-acetic acid 2-methanesulfonylsulfanyl-ethyl ester, 2-propyl-pentanoic acid 2-methanesulfonylsulfanyl-ethyl ester.

As a further object of the present invention is the use of compounds according to the present invention for the preparation of a pharmaceutical composition, and therefore the corresponding method, for preventing, treating or reducing inflammation associated with cardiovascular, connective tissue, pulmonary, gastrointestinal, respiratory, urogenital, nervous or cutaneous systems, infective diseases and tumoral diseases.

It is a further object of the present invention a process for the synthesis of compounds of general formula (I), said process comprising at least:
a) a reaction between the corresponding precursor of an organic thiosulfonate moiety W of formula (II) with a drug D or a corresponding precursor of a drug D modified with X and/or Y, or
b) a reaction between the corresponding precursor of an organic thiosulfonate moiety W of formula (II) modified with X and/or Y, with a drug D,
wherein general formula (I), formula (II), D, X, Y and W have the meaning as stated above.

The compounds of the present invention can be administered in the form of any pharmaceutical formulation, the nature of which will depend upon the route of administration and the nature of the disease to be treated. These pharmaceutical compositions can be prepared by conventional methods, using compatible, pharmaceutically acceptable excipients or vehicles. Examples of such compositions include capsules, tablets, syrups, powders and granulates for the preparation of extemporaneous solutions, injectable preparations, rectal, nasal, ocular, vaginal etc.

A preferred route of administration is the oral route.

The following non-limitative examples further describe and enable an ordinary skilled in the art to make and use the invention.

### EXAMPLE 1. Synthesis of methanethiosulfonic acid S-(2-hydroxyethyl) ester.

A solution of CH₃SO₂Cl (5.9 g) in ethanol (9.2 ml) is added dropwise to a refrigerated (-15°C) solution of Na₂S (46.98 mmol) in ethanol(34.5 ml).

The reaction mixture is stirred at room temperature for 12 hours. After filtration and crystallization from ethanol, sodium methanthiosulfonate, as a white solid is obtained. The sodium methanthiosulfonate (2.5 g; 18.64 mmol) is dissolved in 30 ml of ethanol and a solution of 2-bromoethanol (2.6 ml; 37.28 mmol) in ethanol (6 ml) is added dropwise. The solution is heated at 100 °C for 8 hours under nitrogen. The mixture is filtered, the solution is evaporated to dryness and the residue is dissolved in CHCl₃ and extracted with water.

The aqueous solution is evaporated to dryness, tetrahydrofuran (THF) is added to the residue and the obtained suspension is filtered. The THF solution is evaporated and methanethiosulfonic acid S-(2-hydroxyethyl) ester as an oily yellow product is obtained.

### EXAMPLE 2. Synthesis of 3-methanesulfonylsulfanyl-propionic acid.

Sodium methanthiosulfonate (1.5 g; 11.18 mmol) and 3-bromopropionic acid (900 mg; 5.88 mmol) 97% are dissolved in dimethylformamide (DMF, 9.05 ml). The reaction is performed at 60° C, stirring under nitrogen for 4 hours. The solution is evaporated to dryness and the residue is dissolved in water, acidified with 10 ml of a 2N KHSO₄ and washed with ethyl acetate. The organic phase is extracted with cold 2N KHSO₄, then with brine and finally dried on anhydrous sodium sulphate and evaporated to dryness. An oily yellow product is obtained.

### EXAMPLE 3. Synthesis of 2-acetoxybenzoic acid 2-methanesulfonylsulfanyl-ethyl ester.

Acetyl salicylic acid (691 mg; 3.84 mmol), methanethiosulfonic acid S-(2-hydroxyethyl) ester, prepared as in example 1, (600 mg; 3.84 mmol)and 4-dimethylaminopyridine (DMAP) in catalytic amount (22.5 mg) are dissolved in dichloromethane (67 ml) stirring at room temperature under nitrogen. A solution of 1N dicyclohexylcarbodiimide (DCC; 4.22 mmol, 870.7 mg) in dichloromethane is added and the reaction mixture is stirred for 1.5 hour. At the end of the reaction the dicyclohexylurea is filtered and the solution is extracted successively with a saturated solution of NaHCO₃, water and then 1N HCl. The organic phase is dried on anhydrous sodium sulphate and then evaporated to dryness. The residue is purified by flash chromatography on silica gel using as eluting mixture cycloexhane/ethylacetate (80:20). The product is crystallized with a mixture of ethyl ether/ethyl acetate (9:1) and has a melting point of 90-91°C.

### EXAMPLE 4. Synthesis of {4-[4-ethoxy-3-(1-methyl-7-oxo-3-propyl-6,7-dihydro-1H-pyrazolo[4,3-d]pyrimidin-5-yl)-beazenesulfonyl]-piperazin-1-yl}-acetic acid 2-methanesulfonylsulfanyl-ethyl ester.

The synthesis of 5-{2-ethoxy-5-[4-(hydroxycarbonyl-methyl)piperazinylsulfonyl]-phenyl}-1-methyl-3-n-propyl-1,6-dihydro-7*H*-pyrazolo[4,3-d]pyrimidin-7-one was performed according to Bell, US pat. 5,346,901 and Kim in Biorg. Med. Chem.(2001), 9, 3013-3021. A 1N solution of dicyclohexylcarbodiimide (DCC) (148.5 mg; 0.72 mmol)in dichloromethane is added to a suspension of 5-{2-ethoxy-5-[4-(hydroxycarbonyl-methyl)piperazinylsulfonyl]-phenyl}-1-methyl-3-n-propyl-1,6-dihydro-7H-pyrazolo[4,3-d]pyrimidin-7-one (340 mg; 0.655 mmol), methanethiosulfonic acid S-(2-hydroxyethyl) ester, prepared as in example 1 (102.4 mg; 0.655 mmol)and 4-dimethylaminopyridine (DMAP, 5 mg) in CH₂Cl₂ (30 ml). The reaction is stirred at room temperature, under nitrogen for 3 hours. At the end of the reaction, the dicyclohexylurea is filtered and the solution is extracted with a saturated solution of NaHCO₃. The organic phase is dried on anhydrous sodium sulphate and evaporated to dryness. The residue is purified by chromatography on silica gel using as eluting mixture ethyl acetate with methanol up to 2%. The obtained product, washed with ether and ethanol, has a melting point of 118-120° C.

### EXAMPLE 5. Synthesis of [3-(2,6-dichlorophenylamino)-phenyl]-acetic acid 2methanesulfonylsulfanyl-ethyl ester.

A 1N solution of DCC (581.8 mg; 2.82 mmol) in dichloromethane is added to a solution of diclofenac (758 mg; 2.56 mmol), methanethiosulfonic acid S-(2-hydroxyethyl) ester, prepared as in example 1 (400 mg, 2.56 mmol) and DMAP (15 mg) in dichloromethane (40 ml). The reaction is performed at room temperature, stirring under nitrogen for 1.5 hours. At the end of the reaction the dicyclohexylurea is filtered and the solution is extracted with a saturated solution of NaHCO₃. The organic phase is dried on anhydrous sodium sulphate and evaporated to dryness. The residue is purified by flash chromatography on silica gel using as eluting mixture cyclohexane/ethyl acetate (80:20). The product is crystallized with ether and has a melting point of 41-44° C.

### EXAMPLE 6. Synthesis of 2-propyl-pentanoic acid 2methanesulfonylsulfanyl-ethyl ester.

A solution of methanethiosulfonic acid S-(2-hydroxyethyl) ester, prepared as in example 1 (1.12g; 156.2 mmol) and diisopropylethylamine (1.25 ml) in 3 ml of THF is added dropwise to a solution of valproic acid acyl chloride (1.28 g; 7.17 mmol) in anhydrous tetrahydrofuran (THF, 2 ml). The reaction mixture is stirred at room temperature, under nitrogen for 5 hours. At the end of the reaction the THF is removed and the oily residue is dissolved in CH₂Cl₂ and extracted with 1N HCl, water and sat. NaHCO₃. The organic phase is dried on anhydrous sodium sulphate and evaporated to dryness. The residue is purified by flash chromatography on silica gel using as eluting mixture cyclohexane with ethyl acetate up to 10%. The obtained product is a colourless oil.

### EXAMPLE 7. Biological activity of compound of example 6

The compound described in example 6 has been tested in vitro on 2 prostate tumour cells (DU-145 and PC-3). The compound potently inhibit growth and adhesion to endothelial cells of both prostate tumour cells in a range of concentration of 5-30 µM. The activity reported in the literature for the parent compound (valproic acid) on the same tumour cells is achieved at much higher concentrations (1 mM). (Engl. T et al. Life Sciences, 2005)*.*

### EXAMPLE 8. Biological activity of compound of example 4

Effects of the compound prepared in example 4 on superoxide formation, gp91^{phox} expression by porcine cultured pulmonary endothelial cells and intact cavernosal tissue were studied according to Muzzaffar S. et al. (Br. J. Pharmacol. 2005 Sep;146(1):109-17).
Compound of example 4 was a potent inhibitor of superoxide formation by pulmonary endothelial cells following a 16 hr incubation of pig pulmonary artery cells with TNFα or the thromboxane A₂ analogue, U46619.
Compound of example 4 also inhibited the expression of gp91^{phox} by pulmonary endothelial cells following a 16 hr incubation of pig pulmonary artery cells with TNFα .
These data indicate that the compound of example 4 blocks the expression of NADPH oxidase in response to inflammatory mediators which in turn reduces superoxide formation at lower concentrations than sildenafil citrate.

### EXAMPLE 9. Activity and safety

Thirty male rats (150-200 g) were divided into three groups of 10 animals/group and treated with vehicle, diclofenac (10 mg/kg) and the compound of example 5 (14 mg/kg) for three days once daily by gavage. The vehicle consisted in 1 mL of 0.5% carboxymethylcellulose (CMC). At the fifth day, survival animals were sacrificed, by an overdose of ether, and autopsied to detect any pathological changes, particularly in the gastrointestinal tract. Prostaglandin E2 was evaluated in the liver homogenate by standard enzyme immunoassay procedure as described by Warner TD et al. (J.P.E.T. 2004,310,642-7). The results of this experiment are shown in the following table:

| | % deaths | % gastrointestinal ulcer |
|---|---|---|
| Control | 0 | 0 |
| Diclofenac | 40 | 80 |
| Compound example 5 | 0 | 10 |

Prostanoid formation in liver homogenates of animals treated with compound example 5 or diclofenac was always markedly reduced (by 90% or more) as compared to the samples from vehicle-treated animals. On the whole these results indicate that compound example 5 is significantly better tolerated than diclofenac while maintaining fully the activity (i.e. the ability to inhibit prostaglandin formation).

## Claims

1. Compounds of general formula
D-X-Y-W (I)
wherein D is a drug linked to ~X-Y-W;
X is zero, C=O, COOZ or CONH, wherein Z is a straight or branched C₁-C₄-alkyl ;
Y is zero, -O-(CH₂)ₙ-O- or -OOC-(CH₂)ₙ-COO-, wherein n is 2-10;
W is an organic thiosulfonate moiety having the following formula:
R-S-SO₂-R' (II)
wherein R is -(CH₂)ₙ-COO~; -(CH₂)ₙ-O~; -(CH₂)ₙ-NH~; n = 1-10 linear or branched and it is attached to D-X-Y~; R', straight or branched, is an alkyl, such as methyl, ethyl, propyl, alkenyl, alkinyl, alkylaryl, alkenylaryl, alkinylaryl, arylalkyl, arylalkenyl, arylalkinyl or cycloalkyl, cycloalkenyl, cycloalkinyl, or aromatic and/or heterocyclic ring, all substituted or unsubstituted; and salts thereof.

2. Salts of compounds of general formula (I) according to claim 1, wherein said salts are pharmaceutical acceptable salts such as salts with alkaline metals and alkaline earth metals, non-toxic amines and aminoacids, inorganic acids such as hydrochloric acid, phosphoric acid or organic acids such as fumaric acid, citric acid, tartaric acid.

3. Compound of general formula (I) according to claim 1, wherein D is a drug selected from the group comprising antitumorals such as ancitabine, capecitabine, valproic acid, NSAIDs i.e. selective and unselective cyclo-oxygenase (COX) inhibitors such as ketoprofen, flurbiprofen, suprofen, indobufen, etodolac, diclofenac, indomethacin, naproxen, acetyl salicylic acid, celecoxib, rofecoxib, nimesulide, valdecoxib, parecoxib, lumiracoxib, diflunisal, meloxicam, analgesics such as paracetamol, capsaicin, steroids such as chenodeoxycholic acid, flunisolide, antibiotics such as bacilysin, bronchodilators such as albuterol, expectorants and mucolitic agents such as ambroxol, anti-asthma, antiallergic and anti-histaminic drugs such as epinastine, ACE-inhibitors such as captopril, β-blockers such as atenolol, drugs for vascular disorders suc as brovincamine, antidiabetics such as glibomuride, antiulcer such as omeprazole, arbaprostil, antihyperlipidemics such as fluvastatine, antibacterials such as amoxicilline, antivirals such as amantadine, cGMP phosphodiesterase inhibitors such as sildenafil, verdenafil, drugs against dementia such as mofegiline and the bone reabsorption such as alendronic acid.

4. Compound of general formula (I) according to claim 1, wherein the moiety W is selected from the thiosulfonate group comprising S-(2-carboxyethyl) methanthiosulfonate, S-(2-aminoethyl) methanthiosulfonate and S-(2-hydroxyethyl) methanthiosulfonate.

5. Salts of the compounds of general formula (I) wherein the thiosulfonate moiety is directly linked by an ionic bond to the drug as salt when X=Y=0.

6. Compound of general formula (I) according to claim 1, wherein said compound is 2-acetoxybenzoic acid 2-methanesulfonylsulfanyl-ethyl ester.

7. Compound of general formula (I) according to claim 1, wherein said compound is {4-[4-ethoxy-3-(1-methyl-7-oxo-3-propyl-6,7-dihydro-1H-pyrazolo[4,3-*d*]pyrimidin-5-yl)-benzenesulfonyl]-piperazin-1-yl}-acetic acid 2-methanesulfonyl-sulfanyl-ethyl ester.

8. Compound of general formula (I) according to claim 1, wherein said compound is [3-(2,6-dichlorophenylamino)-phenyl]-acetic acid 2-methanesulfonylsulfanyl-ethyl ester.

9. Compound of general formula (I) according to claim 1, wherein said compound is 2-propyl-pentanoic acid 2-methanesulfonylsulfanyl-ethyl ester.

10. Pharmaceutical composition comprising at least one compound of general formula (I) as claimed in one of the claims 1-9 as an active ingredient and eventually one or more pharmaceutically acceptable adjuvant(s) or carrier(s).

11. Compound of general formula (I) as claimed in one of the claims 1-9 for use as a medicament.

12. Use of a compound of general formula (I) as claimed in one of the claims 1-9 for the preparation of a pharmaceutical composition for preventing, treating or reducing inflammation associated with cardiovascular, connective tissue, pulmonary, gastrointestinal, respiratory, urogenital, nervous or cutaneous systems, infective diseases and tumoral diseases.

13. Process for the synthesis of compounds of general formula (I), said process comprising at least:
a) a reaction between the corresponding precursor of an organic thiosulfonate moiety W of formula (II) with a drug D or a corresponding precursor of a drug D modified with X and/or Y,
or
b) a reaction between the corresponding precursor of an organic thiosulfonate moiety W of formula (II) modified with X and/or Y, with a drug D,
wherein general formula (I), formula (II), D, X, Y and W have the meaning according to claim 1.
